# EUROPEAN PATENT SPECIFICATION

(11) **EP 4 014 932 B1**
(45) Date of publication and mention of the grant of the patent: **06.09.2023**
(21) Application number: 21163798.8
(22) Date of filing: 19.03.2021
(51) Int. Cl.: A61F 2/66, A61F 2/76, A61F 2/50

(54) **MULTI-AXIS ANKLE JOINT PROSTHESIS**
MEHRACHSIGE SPRUNGGELENKPROTHESE
PROTHÈSE D'ARTICULATION DE CHEVILLE À AXES MULTIPLES

(30) Priority: 16.12.2020 CN 202011482844
(43) Date of publication of application: 22.06.2022
(73) Proprietor: Jilin University, Changchun, Jilin 130012 (CN)
(72) Inventor: REN, Lei, Changchun City, Jilin (CN); WANG, Kunyang, Changchun City, Jilin (CN); ZENG, Yi, Changchun City, Jilin (CN); QIAN, Zhihui, Changchun City, Jilin (CN); XIU, Haohua, Changchun City, Jilin (CN); LIANG, Wei, Changchun City, Jilin (CN); REN, Luquan, Changchun City, Jilin (CN)
(74) Representative: Leihkauf, Steffen Falk

(56) References cited:
- EP-A1- 3 219 295
- CN-A- 102 940 542
- CN-A- 111 714 259
- US-A1- 2005 261 783
- US-A1- 2005 267 600
- US-A1- 2006 247 794

## Description

### Cross-Reference to Related Application

The present invention claims priority to Chinese Patent Application No. 202011482844.6, filed on December 16, 2020 and entitled "Multi-axis ankle joint prosthesis with adjustable joint axis angle and spherical structure".

### Technical Field

The present invention belongs to the technical field of human body prostheses, and particularly relates to a multi-axis ankle joint prosthesis with an adjustable joint axis angle and a spherical structure.

### Background

At present, ankle joint prosthetic products involved in the market are mainly square in appearance, which are not natural and attractive enough for a wearer, ankle joint prostheses all take axes as main force bearing elements, and however, force generated in a walking process of people is very large, usually resulting in a large shaft diameter, and a large overall size of the prostheses; a human body ankle joint axis consists of multiple axes for better improving the use experience of the wearer, the multi-axis joint prostheses are a mainstream research direction at present; for some multi-axis ankle joint prosthetic products on the market, a space angle of two axes of the prosthesis is fixed, and for different amputated patients, ankle joint angles of healthy limbs of the patients are different, such that the personalized adaptability to the product is low, the movement coordination between an affected side limb and a normal side limb of the wearer is poor, the gait is uncoordinated, and the adaptability to different road surfaces is poor.

A joint axis angle adjustable type multi-axis ankle joint prosthesis is disclosed in D1 (CN 111714259 A);
An artificial limb ankle is disclosed in D2 (CN 102940542 A);
An alignment assembly for a modular prosthesis is disclosed in D3 (US 2005/267600 A1);
A multi-axis prosthetic ankle for connection of a prosthetic lower leg to a prosthetic foot is disclosed in D4 (US 2006/247794 A1);
A fitting for a prosthetic foot permits multi-axial movement of the prosthetic foot with respect to a lower limb prosthesis and provides shock absorption is disclosed in D5 (US 2005/261783 A1).

### Summary

The invention is set out in the appended set of claims.

The present invention provides a multi-axis ankle joint prosthesis product with an adjustable joint axis angle and a spherical structure, as for the prosthesis product, movement around an axis is replaced with movement along a rail, an overall size is reduced, a structure is simple, bearing capacity is high, multi-axis combination is achieved, a wearer can carry out personalized adjustment on a space angle direction of an ankle joint movement axis by means of simple operation, accordingly, the movement coordination between an affected side limb and a normal side limb of the wearer is improved, the environmental adaptability and the wearing comfort of the prosthesis are improved, and different road surface changes can be coped.

The present invention includes a foot connection part A, a leg connection part B, an upper part C, a lower part D, a first ball 1, a second ball 2, a third ball 3 and an auxiliary plate 42, wherein the foot connection part A, the lower part D, the upper part C and the leg connection part B are sequentially arranged from bottom to top; a first boss 22 of a slide block assembly I E in the upper part C is located in a first elongated slide groove f of an upper cover 7 in the leg connection part B, and the first boss 22 is in clearance fit with the first elongated slide groove f; a first threaded rod 21 of the first slide block assembly E in the upper part C passes through the first elongated slide groove f of the upper cover 7 in the leg connection part B and is fixedly connected by a nut; a second boss 36 of a second slide block assembly G in the lower part D is located in a second elongated slide groove b of a lower cover 5 in the foot connection part A, and the second boss 36 is in clearance fit with the second elongated slide groove b; a second threaded rod 37 of the second slide block assembly G in the lower part D passes through the second elongated slide groove b of the lower cover 5 in the foot connection part A and is fixedly connected by means of a nut; a sphere 32 of a sphere assembly H in the lower part D is placed in a hemispherical shell 16 of a hemispherical shell assembly F in the upper part C, and the sphere 32 is clamped and fixedly connected by screwing a bolt 18 of the hemispherical shell assembly F; the first ball 1 is located between the foot connection part A and the lower part D, the first ball 1 makes contact with a lower surface of the sphere 32 of the sphere assembly H in the lower part D, and the first ball 1 is limited by a first local spherical pit a of the lower cover 5 in the foot connection part A; the second ball 2 is located between the leg connection part B and the upper part C, the second ball 2 makes contact with the hemispherical shell 16 of the hemispherical shell assembly F in the upper part C, and the second ball 2 is limited by a second local spherical pit e of the upper cover 7 in the leg connection part B; and the third ball 3 is located between the leg connection part B and the upper part C, the third ball 3 makes contact with the hemispherical shell 16 of the hemispherical shell assembly F in the upper part C, and the third ball 3 is limited by a third local spherical pit g of the upper cover 7 in the leg connection part B. The auxiliary plate 42 is placed between the foot connection part A and the leg connection part B during angle adjustment.

The foot connection part A includes a first connector 4 and the lower cover 5, wherein the lower cover 5 is of a hollow hemisphere shape with an opening facing upwards, a first stand column 6 is arranged in a center of a lower end of the lower cover 5, and a first threaded blind hole c is provided in a center of a lower end of the first stand column 6; the second elongated slide groove b is provided in a middle of a right side of the lower cover 5, the first local spherical pit a is provided in an inner wall of the lower cover 6, and the first local spherical pit a is located on a longitudinal extending line of the second elongated slide groove b; a first through hole d is provided in a center of the first connector 4; and the first connector 4 is fixedly connected with the lower end of the first stand column 6 of the lower cover 5 by means of a bolt.

The leg connection part B includes the upper cover 7 and a second connector 8, wherein the upper cover 7 is in a hollow hemisphere shape with an opening facing downwards, a second stand column 9 is arranged in a center of an upper end of the upper cover 7, and a second threaded blind hole i is provided in a center of an upper end of the second stand column 9; a notch h is provided in a lower end of a left side of the upper cover 7, and the first elongated slide groove f is provided in a right side of the upper cover 7; the second local spherical pit e and the third local spherical pit g are symmetrically provided in a front side and a rear side of an inner wall of the upper cover 7; a second through hole j is provided in a center of the second connector 8; and the second connector 8 is fixedly connected to the upper end of the second stand column 9 of the upper cover 7 by means of a bolt.

The upper part C includes a first limiting block 10, the first slide block assembly E, a second limiting block 11, the hemispherical shell assembly F, a first spring 12 and a second spring 13, wherein the first slide block assembly E includes the first threaded rod 21, the first boss 22 and a first protruding block 23, the first threaded rod 21, the first boss 22 and the first protruding block 23 are arranged from top to bottom and being fixedly connected; the hemispherical shell assembly F includes a first vertical plate 14, a second vertical plate 15, the hemispherical shell 16, a rod pair 17 and a bolt 18, a first notch 19 is provided in a lower surface of a right side of the hemispherical shell 16, a left notch 20 is provided in the middle of the left side of the hemispherical shell 16, right ends of two rods of the rod pair 17 are fixedly connected with a lower surface of a left side of the hemispherical shell 16 and a front side and a rear side of the left notch 20, left ends of the two rods of the rod pair 17 are connected by means of the bolt 18, and a maximum radian of the hemispherical shell 16 slightly exceeds a sphere center; the first vertical plate 14 and the second vertical plate 15 are arranged in parallel front and back, and an inverted-T-shaped rail is formed between the first vertical plate 14 and the second vertical plate 15; lower surfaces of the first vertical plate 14 and the second vertical plate 15 are fixedly connected with an upper surface of a right part of the hemispherical shell 16; the first limiting block 10 includes a first transverse block 24 and a first vertical block 25, a lower surface of a right part of the first transverse block 24 is fixedly connected with a top end of the first vertical block 25; the second limiting block 11 includes a second transverse block 26 and a second vertical block 27, a lower surface of a left part of the second transverse block 26 is fixedly connected with a top end of the second vertical block 27; the first limiting block 10, the first spring 12, the first protruding block 23 of the first slide block assembly E, the second spring 13 and the second limiting block 11 are sequentially arranged from right to left and located between the first vertical plate 14 and the second vertical plate 15; the first vertical block 25 of the first limiting block 10 is fixedly connected with lower right ends of the first vertical plate 14 and the second vertical plate 15; the second vertical block 27 of the second limiting block 11 is fixedly connected with upper left ends of the first vertical plate 14 and the second vertical plate 15; and the first protruding block 23 of the first slide block assembly E is in sliding connection to the inverted-T-shaped rail between the first vertical plate 14 and the second vertical plate 15.

The lower part D includes the second slide block assembly G, a third limiting block 28, the sphere assembly H, a fourth limiting block 29, a third spring 30 and a fourth spring 31, wherein the second slide block assembly G includes a second protruding block 35, the second boss 36 and a second threaded rod 37, the second protruding block 35, the second boss 36 and the second threaded rod 37 being arranged from bottom to top and fixedly connected; the sphere assembly H includes the sphere 32, a third vertical plate 33 and a fourth vertical plate 34, the third vertical plate 33 and the fourth vertical plate 34 being arranged in parallel left and right, and the inverted-T-shaped rail being formed between the third vertical plate 33 and the fourth vertical plate 34; lower surfaces of the third vertical plate 33 and the fourth vertical plate 34 are fixedly connected to an upper surface of the right part of the sphere 32; the third limiting block 28 includes a third transverse block 38 and a third vertical block 39, the third transverse block 38 being fixedly connected with a top end of the third vertical block 39; the fourth limiting block 29 includes a fourth transverse block 40 and a fourth vertical block 41, the fourth transverse block 40 is fixedly connected with a top end of the fourth vertical block 41; the third limiting block 28, the third spring 30, the second protruding block 35 of the second slide block assembly G, the fourth spring 31 and the fourth limiting block 29 are sequentially arranged from front to back and located between the third vertical plate 33 and the fourth vertical plate 34; the third vertical block 39 of the third limiting block 28 is fixedly connected with rear ends of the third vertical plate 33 and the fourth vertical plate 34; the fourth vertical block 41 of the fourth limiting block 29 is fixedly connected with front ends of the third vertical plate 33 and the fourth vertical plate 34; and the second protruding block 35 of the second slide block assembly G is in sliding connection to the inverted-T-shaped rail between the third vertical plate 33 and the fourth vertical plate 34.

A joint axis angle adjustment process of the present invention is as follows:
1. placing the auxiliary plate 42 between the foot connection part A and the leg connection part B;
2. loosening the bolt 18;
3. loosening the nut on the first threaded rod 21;
4. keeping the foot connection part A stationary, horizontally rotating the leg connection part B to drive the upper part C to horizontally rotate to a proper position together, and equivalently, completing adjustment of a horizontal angle of an ankle joint axis;
5. rotating the first slide block assembly E along the first elongated slide groove f to drive the upper part C to rotate to a proper position along the first elongated slide groove f, and equivalently, completing adjustment of a vertical angle of the ankle joint axis;
6. locking the nut on the first threaded rod 21, and equivalently, completing angle adjustment of the ankle joint axis;
7. loosening the nut on the second threaded rod 37;
8. keeping the leg connection part B stationary, horizontally rotating the foot connection part A to drive the lower part D to horizontally rotate to a proper position together, and equivalently, completing adjustment of a horizontal angle of a subtalar joint axis;
9. rotating the second slide block assembly G along the second elongated slide groove b to drive the lower part D to rotate to a proper position along the second elongated slide groove b, and equivalently, completing adjustment of a vertical angle of the subtalar joint axis;
10. locking the nut on the second threaded rod 37, and equivalently, completing angle adjustment of the subtalar joint axis;
11. screwing the bolt 18 to make the hemisphere shell 16 clamp the sphere 32; and
12. taking the auxiliary plate 42 out.

So far, the adjustment of the joint axis angle is completed.

Some embodiments of the present invention have the beneficial effects:
As for the shape, a sphere shape is used, which is more attractive and close to the ankle joint of a human body; as for movement, sliding along a rail is used to replace traditional rotation around a shaft, the sphere is used for stress to replace a previous stress mode of the shaft, which greatly improves the bearing capacity; as for angle adjustment, the design is simpler and more convenient, and the wearer can adjust the angle of the joint axis only by rotating an external component; without shaft components, the components playing a role in angle adjustment are replaced with spherical shell covers, the number of the needed components is small, and the structure is very simple; and before use, a joint angle of an ankle joint on one the of healthy limb of the human body is measured firstly, and then an angle of the joint axis of the prosthesis product is automatically adjusted until the optimal wearing and using effect is achieved. According to the present invention, the problems that an ankle joint prosthesis product is complex in structure, low in matching degree, poor in adaptability and large in energy consumption of a wearer are solved to a large extent.

### Brief Description of the Drawings

The accompanying drawings, forming a part of the present application, of the description serve to provide a further understanding of the present invention, and the illustrative embodiments of the present invention and the description of the illustrative embodiments serve to explain the present invention and are not to be construed as unduly limiting the present invention. In the drawings:
Fig. 1 illustrates a perspective view of a multi-axis ankle joint prosthesis with an adjustable joint axis angle and a spherical structure;
Fig. 2 illustrates a sectional view of the multi-axis ankle joint prosthesis with the adjustable joint axis angle and the spherical structure;
Fig. 3 illustrates a perspective view of a foot connection part A of the multi-axis ankle joint prosthesis in Fig. 1;
Fig. 4 illustrates a perspective top view of a lower cover of the multi-axis ankle joint prosthesis in Fig. 1;
Fig. 5 illustrates a perspective bottom view of the lower cover in Fig.4;
Fig. 6 illustrates a sectional view of the lower cover of the multi-axis ankle joint prosthesis in Fig. 1;
Fig. 7 illustrates a perspective view of a first connector;
Fig. 8 illustrates a perspective view of a leg connection part;
Fig. 9 illustrates a perspective top view of an upper cover;
Fig. 10 illustrates a perspective bottom view of the upper cover;
Fig. 11 illustrates a sectional view of the upper cover;
Fig. 12 illustrates a perspective view of a second connector;
Fig. 13 illustrates a perspective view of an upper part;
Fig. 14 illustrates a sectional view of the upper part;
Fig. 15 illustrates a first perspective view of a hemispherical shell assembly;
Fig. 16 illustrates a second perspective view of the hemispherical shell assembly;
Fig. 17 illustrates a perspective view of a first slide block assembly;
Fig. 18 illustrates a perspective view of a first limiting block;
Fig. 19 illustrates a perspective view of a second limiting block;
Fig. 20 illustrates a perspective view of a lower part;
Fig. 21 illustrates a sectional view of the lower part ;
Fig. 22 illustrates a perspective view of a sphere assembly;
Fig. 23 illustrates a perspective view of a second slide block assembly;
Fig. 24 illustrates a perspective view of a third limiting block;
Fig. 25 illustrates a perspective view of a fourth limiting block;
Fig. 26 illustrates a perspective view of the multi-axis ankle joint prosthesis with the adjustable joint axis angle and the spherical structure during auxiliary plate installation; and
Fig. 27 illustrates a perspective view of the auxiliary plate.

The above-mentioned figures comprise the following reference numerals:
A. foot connection part; B. leg connection part; C. upper part; D. lower part E. first slide block assembly; F. hemispherical shell assembly; G. second slide block assembly; H. sphere assembly; 1. first ball; 2. second ball; 3. third ball; 4. first connector; 5. lower cover; 6. first stand column; 7. upper cover; 8. second connector; 9. second stand column; 10. first limiting block; 11. second limiting block; 12. first spring; 13. second spring; 14. first vertical plate; 15. second vertical plate; 16. hemispherical shell; 17. rod pair; 18. bolt; 19. first notch; 20. left notch; 21, first threaded rod; 22. first boss; 23. a first protruding block; 24. first transverse block; 25. first vertical block; 26. second transverse block; 27. second vertical block; 28. third limiting block; 29. fourth limiting block; 30. third spring; 31. fourth spring 32. sphere; 33. third vertical plate; 34. fourth vertical plate; 35. second protruding block; 36.second boss; 37. second threaded rod; 38. third transverse block; 39. third vertical block; 40. fourth transverse block; 41. fourth vertical block; 42. auxiliary plate; a. first local spherical pit; b. second elongated slide groove; c. first threaded blind hole; d. first through hole; e. second local spherical pit ; f. first elongated slide groove; g. third local spherical pit ; h. notch; i. second threaded blind hole; j. second through hole.

### Detailed Description of the Embodiments

It should be noted that the embodiments of the present application and the features of the embodiments may be combined with each other without conflict. The present invention will be described in detail below with reference to the accompanying drawings in conjunction with embodiments. The present invention will be described hereafter in conjunction with the accompanying drawings.

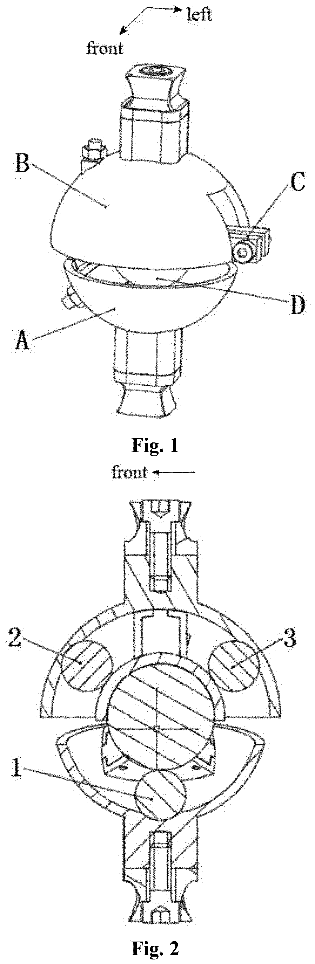

As shown in Figs. 1, 2, 26 and 27, the present invention illustrates a foot connection part A, a leg connection part B, an upper part C, a lower part D, a first ball 1, a second ball 2, a third ball 3 and an auxiliary plate 42, wherein the foot connection part A, the lower part D, the upper part C and the leg connection part B are sequentially arranged from bottom to top; a first boss 22 of a first slide block assembly E in the upper part C is located in a first elongated slide groove f of an upper cover 7 in the leg connection part B, and the first boss 22 is in clearance fit with the first elongated slide groove f; a first threaded rod 21 of the first slide block assembly E in the upper part C passes through the first elongated slide groove f of the upper cover 7 in the leg connection part B and is fixedly connected by means of a nut; a second boss 36 of a second slide block assembly G in the lower part D is located in a second elongated slide groove b of a lower cover 5 in the foot connection part A, and the second boss 36 is in clearance fit with the second elongated slide groove b; a second threaded rod 37 of the second slide block assembly G in the lower part D passes through the second elongated slide groove b of the lower cover 5 in the foot connection part A and is fixedly connected by a nut; a sphere 32 of a sphere assembly H in the lower part D is placed in a hemispherical shell 16 of a hemispherical shell assembly F in the upper part C, and the sphere 32 is clamped and fixedly connected by screwing a bolt 18 of the hemispherical shell assembly F; the first ball 1 is located between the foot connection part A and the lower part D, the first ball 1 contacts with a lower surface of the sphere 32 of the sphere assembly H in the lower part D, and the first ball 1 is limited by a first local spherical pit a of the lower cover 5 in the foot connection part A; the second ball 2 is located between the leg connection part B and the upper part C, the second ball 2 contacts with the hemispherical shell 16 of the hemispherical shell assembly F in the upper part C, and the second ball 2 is limited by a second local spherical pit e of the upper cover 7 in the leg connection part B; and the third ball 3 is located between the leg connection part B and the upper part C, the third ball 3 makes contact with the hemispherical shell 16 of the hemispherical shell assembly F in the upper part C, and the third ball 3 is limited by a third local spherical pit g of the upper cover 7 in the leg connection part B. The auxiliary plate 42 is placed between the foot connection part A and the leg connection part B during angle adjustment.

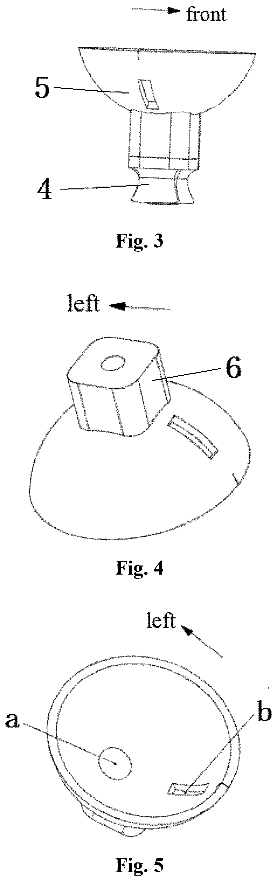

As shown in Figs. 3-7, in some embodiments, the foot connection part A includes a first connector 4 and the lower cover 5, wherein the lower cover 5 is of a hollow hemisphere shape with an opening facing upwards, a first stand column 6 is arranged in a center of a lower end of the lower cover 5, and a first threaded blind hole c is provided in a center of a lower end of the first stand column 6; the second elongated slide groove b is provided in a middle of a right side of the lower cover 5, the first local spherical pit a is provided in an inner wall of the lower cover 6, and the first local spherical pit a is located on a longitudinal extending line of the second elongated slide groove b; a first through hole d is provided in a center of the first connector 4; and the first connector 4 is fixedly connected with the lower end of the first stand column 6 of the lower cover 5 by means of a bolt.

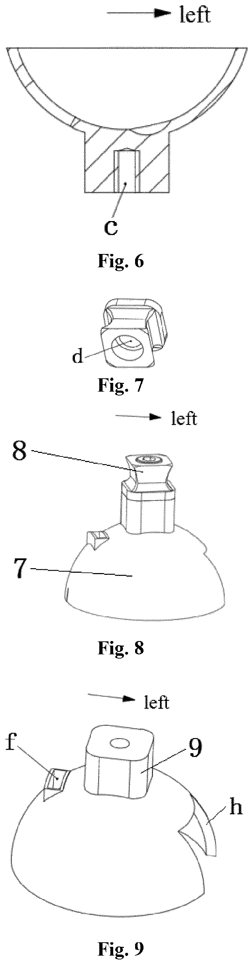

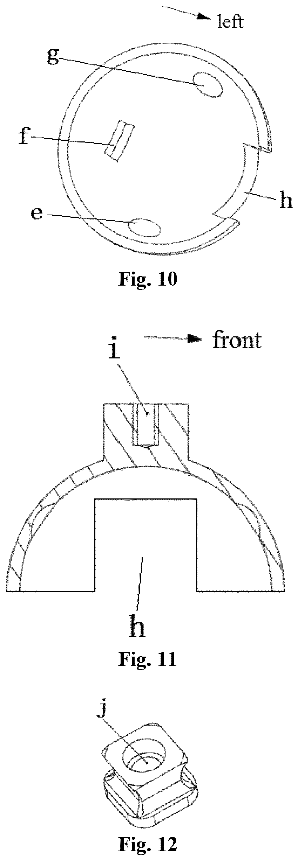

As shown in Figs. 8-12, in some embodiments, the leg connection part B includes the upper cover 7 and a second connector 8, wherein the upper cover 7 is in a hollow hemisphere shape with an opening facing downwards, a second stand column 9 is arranged in a center of an upper end of the upper cover 7, and a second threaded blind hole i is provided in a center of an upper end of the second stand column 9; a notch h is provided in a lower end of a left side of the upper cover 7, and the first elongated slide groove f is provided in a right side of the upper cover 7; the second local spherical pit e and the third local spherical pit g are symmetrically provided in a front side and a rear side of an inner wall of the upper cover 7; a second through hole j is provided in a center of the second connector 8; and the second connector 8 is fixedly connected with the upper end of the second stand column 9 of the upper cover 7 by means of a bolt.

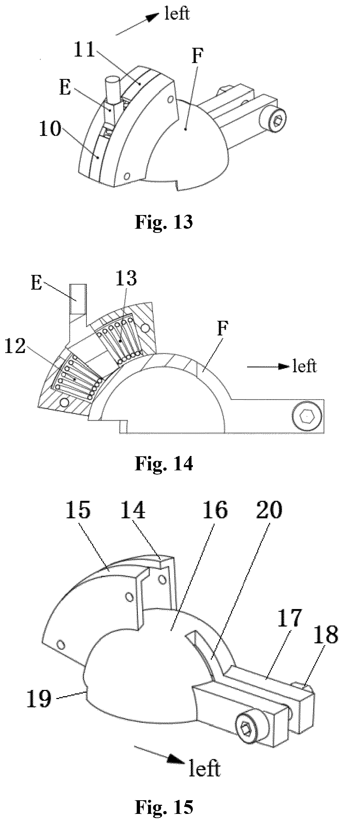

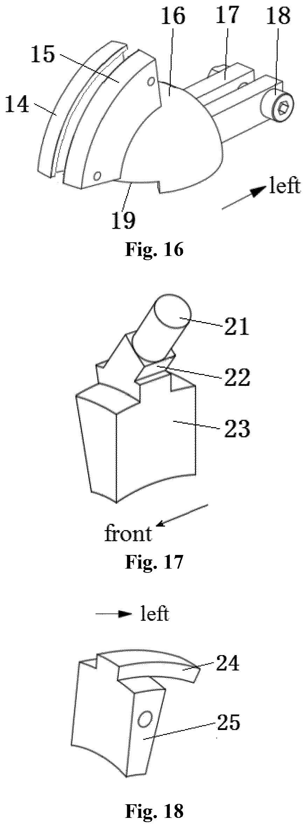

As shown in Figs. 13-19, in some embodiments, the upper part C includes a first limiting block 10, the first slide block assembly E, a second limiting block 11, the hemispherical shell assembly F, a first spring 12 and a second spring 13, wherein the first slide block assembly E consists of the first threaded rod 21, the first boss 22 and a first protruding block 23, the first threaded rod 21, the first boss 22 and the first protruding block 23 are arranged from top to bottom and being fixedly connected; the hemispherical shell assembly F includes a first vertical plate 14, a second vertical plate 15, the hemispherical shell 16, a rod pair 17 and a bolt 18, a first notch 19 is provided in a lower surface of a right side of the hemispherical shell 16, a left notch 20 is provided in a middle of a left side of the hemispherical shell 16, right ends of two rods of the rod pair 17 are fixedly connected with a lower surface of a left side of the hemispherical shell 16 and a front side and a rear side of the left notch 20, left ends of the two rods of the rod pair 17 are connected by means of the bolt 18, and a maximum radian of the hemispherical shell 16 slightly exceeds a sphere center; the first vertical plate 14 and the second vertical plate 15 are arranged in parallel front and back, and an inverted-T-shaped rail is formed between the first vertical plate 14 and the second vertical plate 15; lower surfaces of the first vertical plate 14 and the second vertical plate 15 are fixedly connected with an upper surface of a right part of the hemispherical shell 16; the first limiting block 10 includes a first transverse block 24 and a first vertical block 25, a lower surface of a right part of the first transverse block 24 are fixedly connected with a top end of the first vertical block 25; the second limiting block 11 includes a second transverse block 26 and a second vertical block 27, a lower surface of a left part of the second transverse block 26 are fixedly connected with a top end of the second vertical block 27; the first limiting block 10, the first spring 12, the first protruding block 23 of the first slide block assembly E, the second spring 13 and the second limiting block 11 are sequentially arranged from right to left and located between the first vertical plate 14 and the second vertical plate 15; the first vertical block 25 of the first limiting block 10 is fixedly connected with lower right ends of the first vertical plate 14 and the second vertical plate 15; the second vertical block 27 of the second limiting block 11 is fixedly connected with upper left ends of the first vertical plate 14 and the second vertical plate 15; and the first protruding block 23 of the first slide block assembly E is in sliding connection to the inverted-T-shaped rail between the first vertical plate 14 and the second vertical plate 15.

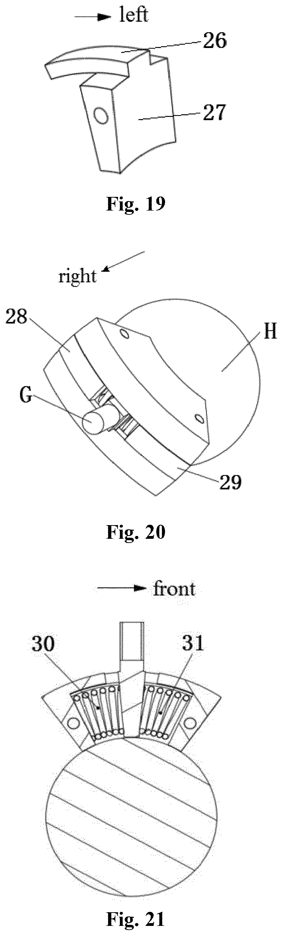

As shown in Figs. 20-25, in some embodiments, the lower part D includes the second slide block assembly G, a third limiting block 28, the sphere assembly H, a fourth limiting block 29, a third spring 30 and a fourth spring 31, wherein the second slide block assembly G includes a second protruding block 35, the second boss 36 and a second threaded rod 37, the second protruding block 35, the second boss 36 and the second threaded rod 37 are arranged from bottom to top and fixedly connected; the sphere assembly H includes the sphere 32, a third vertical plate 33 and a fourth vertical plate 34, the third vertical plate 33 and the fourth vertical plate 34 are arranged in parallel left and right, and the inverted-T-shaped rail is formed between the third vertical plate 33 and the fourth vertical plate 34; lower surfaces of the third vertical plate 33 and the fourth vertical plate 34 are fixedly connected with an upper surface of the right part of the sphere 32; the third limiting block 28 includes a third transverse block 38 and a third vertical block 39, the third transverse block 38 is fixedly connected with a top end of the third vertical block 39; the fourth limiting block 29 includes a fourth transverse block 40 and a fourth vertical block 41, the fourth transverse block 40 is fixedly connected with a top end of the fourth vertical block 41; the third limiting block 28, the third spring 30, the second protruding block 35 of the second slide block assembly G, the fourth spring 31 and the fourth limiting block 29 are sequentially arranged from front to back and located between the third vertical plate 33 and the fourth vertical plate 34; the third vertical block 39 of the third limiting block 28 is fixedly connected with rear ends of the third vertical plate 33 and the fourth vertical plate 34; the fourth vertical block 41 of the fourth limiting block 29 is fixedly connected with front ends of the third vertical plate 33 and the fourth vertical plate 34; and the second protruding block 35 of the second slide block assembly G is in sliding fit with the inverted-T-shaped rail between the third vertical plate 33 and the fourth vertical plate 34.

As shown in Figs. 1, 5, 9, and 13-22, the embodiments of the present invention provide a multi-axis ankle joint prosthesis. The multi-axis ankle joint prosthesis of the embodiment includes a leg connection part B, a foot connection part A, an upper part C and a lower part D, wherein the leg connection part B includes an upper cover 7 and a first elongated slide groove provided in the upper cover 7; the foot connection part A includes a lower cover 5 and a second elongated slide groove b provided in the lower cover 5; the upper part C includes a hemispherical shell assembly F located in the upper cover 7 and a first slide block assembly E at least partially located in the upper cover, the first slide block assembly E includes a first boss 22 in sliding fit with the first elongated slide groove f and a first threaded rod 21 connected with the first boss 22, wherein the first threaded rod 21 passes through the first elongated slide groove f and is detachably installed on the upper cover 7 by a first locking member; the hemispherical shell assembly F includes a hemispherical shell 16, and the leg connection part B and the first slide block assembly E are rotatably arranged relative to the hemispherical shell 16; and the lower part D includes a sphere assembly H and a second slide block assembly G which are located in the lower cover 5, the second slide block assembly G includes a second boss 36 in sliding fit with the second elongated slide groove b and a second threaded rod 37 connected with the second boss 36, wherein one end of the second threaded rod 37 penetrates out of the second elongated slide groove b and is detachably installed on the lower cover 5 by a second locking member, the sphere assembly H includes a sphere 32 located in the hemisphere shell 16, wherein the sphere 32 is connected with the hemisphere shell 16, and the foot connection part A and the second slide block assembly G are rotatably arranged relative to the sphere 32.

By means of the above arrangement, due to the fact that the first boss 22 is able to slide along the first elongated slide groove f, a position of the first slide block assembly E relative to the upper cover 7 is adjusted according to actual requirements; similarly, due to the fact that the second boss 36 is able to slide along the second elongated slide groove b, a position of the second slide block assembly G relative to the lower cover 5 may be adjusted according to actual conditions, thus, adjustment may be conducted according to the actual conditions of different wearers, and the multi-axis ankle joint prosthesis is good in adaptability and wider in application range.

Specifically, the first locking member is loosened, when external force acts on the first threaded rod 21, the first boss 22 is driven to slide along the first elongated slide groove f, and when the first boss 22 slides to a certain position, the first slide assembly E is fixed to the upper cover 7 by means of the first locking member, such that the position of the first slide block assembly E relative to the upper cover 7 is adjusted, that is, a function of adjusting the position of the upper part C relative to the upper cover 7 is achieved; similarly, when external force acts on the second threaded rod 37, the second boss 36 is able to be driven to slide along the second elongated slide groove b, when the second boss 36 slides to a needed position, the second slide block assembly G is fixed to the lower cover by the second locking member, such that the position of the second slide block assembly G relative to the lower cover 5 is adjusted, and a function of adjusting the position of the lower part D relative to the lower cover 5 is achieved, thereby achieving angle adjustment of the joint axis from different directions, and the joint prosthesis may be attached to a joint angle of an ankle joint on the side of healthy limb of the human body as much as possible.

In one embodiment, the first locking member and the second locking member are both nuts for convenient disassembly and assembly.

In addition, as shown in Figs. 1, 17, and 23, the first threaded rod 21 is detachably installed on the upper cover 7, the second threaded rod 37 is detachably installed on the lower cover 5, the first threaded rod 21 is located outside the upper cover 7, and the second threaded rod 37 is located outside the lower cover 5, such that it is possible for the wearer to adjust by himself/herself.

According to the present invention, as shown in Figs. 14-17, the first slide block assembly E further includes a first protruding block 23 connected with the first boss 22, and the hemispherical shell assembly F further includes a first rail arranged on the hemispherical shell 16, the first protruding block 23 is in sliding fit with the first rail, so as to make the leg connection part B and the first protruding block 23 rotate around a sphere center of the hemispherical shell 16.

Compared with rotation around a shaft in the related art, the bearing capacity is greatly improved by means of the spherical-like hemispherical shell 16 and a sliding mode along the rail, the mode that the leg connection part B and the first protruding block 23 rotate around the sphere center of the hemispherical shell 16 is closer to the ankle joint of the human body, thereby improving the comfort and the using effect of the wearer. Further, a spherical structure is used, the overall size is small, and the number of parts is small.

According to the present invention, as shown in Figs. 20-23, the second slide block assembly G further includes a second protruding block 35 connected with the second boss 36, and the sphere assembly H further includes a second rail arranged on an outer surface of the sphere 32, the second protruding block 35 is in sliding fit with the second rail, so as to make the foot connection part A and the second protruding block 35 rotate around a sphere center of the sphere 32.

In the above arrangement, the second protruding block 35 is in sliding fit with the second rail, so as to make the foot connection part A and the second protruding block 35 rotate around the sphere center of the sphere 32. By using the spherical structure and a sliding fit mode, the movement of the ankle joint prosthesis is closer to that of the ankle joint of the human body, thereby improving the comfort of the wearer.

In some embodiments, the sphere center of the sphere 32 coincides with the sphere center of the hemispherical shell 16. In this way, the movement of the ankle joint prosthesis may be closer to that of the ankle joint of the healthy limb of a person, thereby improving the use experience of the wearer.

In an embodiment of the present invention, as shown in Fig. 2, the multi-axis ankle joint prosthesis further includes: a first ball 1, located between the lower cover 5 and the sphere 32; a second ball 2, located between the upper cover 7 and the hemispherical shell 16; and a third ball 3, located between the upper cover 7 and the hemispherical shell 16, the second ball 2 and the third ball 3 are arranged in a front-back direction; and wherein an inner wall of the lower cover is provided with a first local spherical pit used for limiting the first ball, and the upper cover is provided with a second local spherical pit used for limiting the second ball and a third local spherical pit used for limiting the third ball.

By means of the above arrangement, on one hand, it can be guaranteed that the first slide block assembly E and the leg connection part B rotate more smoothly around the sphere center of the hemisphere shell 16 in a first direction (that is, a left-right direction shown in Fig. 15), and on the other hand, it can be guaranteed that the second slide block assembly G and the foot connection part A rotate more smoothly around the sphere center of the sphere 32 in a second direction (that is, a front-back direction shown in Fig. 21).

According to the present invention, as shown in Fig. 15, the hemispherical shell assembly further include a first vertical plate 14 and a second vertical plate 15 arranged in parallel front and back on a surface of the hemispherical shell 16, a first rail is formed between the first vertical plate 14 and the second vertical plate 15.

In some embodiments, the first rail is an arc-shaped rail extending in a left-right direction along the surface of the hemispherical shell 16; the first rail is simple in structure.

In an embodiment of the present invention, as shown in Fig. 13, the hemispherical shell assembly F further includes a first limiting block 10 connected with the first vertical plate 14 and a second limiting block 11 connected with the second vertical plate 15, the first protruding block 23 is located between the first limiting block 10 and the second limiting block.

By means of the above arrangement, a limit position of the movement of the first protruding block 23 in the first rail is limited, such that the use experience of the ankle joint prosthesis is better.

As shown in Fig. 14, in an embodiment of the present invention, for guaranteeing a relatively smooth movement of the first protruding block 23, the multi-axis ankle joint prosthesis further includes a first elastic assembly located in the first rail. The first elastic assembly comprises a first spring 12 and a second spring 13, the first spring 12 is located between the first limiting block 10 and the first protruding block 23, the second spring 13 is located between the first protruding block 23 and the second limiting block 11, thus, the first limiting block 10, the first spring 12, the first protruding block 23, the second spring 13 and the second limiting block 11 may be sequentially arranged from left to right.

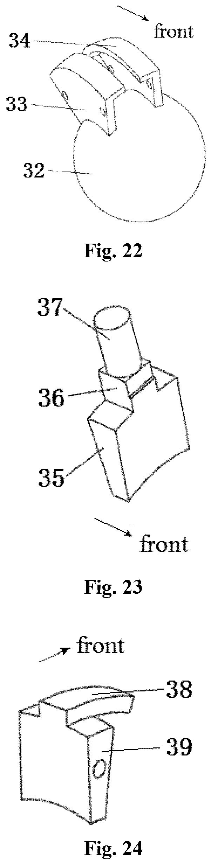

In an embodiment of the present invention, as shown in Fig. 22, the sphere assembly H further comprises a third vertical plate 33 and a fourth vertical plate 34 arranged in parallel left and right on a surface of the sphere 32, the second rail is formed between the third vertical plate 33 and the fourth vertical plate 34.

By means of the above arrangement, the second protruding block 35 may slide on the surface of the sphere 32 along the second rail, the sliding mode is closer to that of the ankle joint of the healthy limb of the human body, and the user experience is good.

In some exemplary embodiments, the second rail is an arc-shaped track extending in a front-back direction along the surface of the sphere 32.

In an embodiment of the present invention, as shown in Figs. 20-22, for limiting a limit position of the movement of the second protruding block 35, the sphere assembly H further includes a third limiting block 28 arranged at a first end of the third vertical plate 33 and a fourth limiting block 29 arranged at a second end of the third vertical plate 33, the second protruding block 35 is located between the third limiting block 28 and the fourth limiting block 29.

As shown in Fig. 21, in an embodiment of the present invention, for guaranteeing a relatively smooth movement of the second protruding block 35, the multi-axis ankle joint prosthesis further includes a second elastic assembly located in the second rail. The second elastic assembly includes a third spring 30 and a fourth spring 31, the third spring 30 is located between the third limiting block 28 and the second protruding block 35, the fourth spring 31 is located between the second protruding block 35 and the fourth limiting block 29, thus, the third limiting block 28, the third spring 30, the second protruding block 35, the fourth spring 31 and the fourth limiting block 29 may be sequentially arranged from front to back.

In an embodiment of the present invention, as shown in Fig. 15, a left notch 20 is provided in the hemispherical shell 16, the hemispherical shell assembly further includes a rod pair 17, wherein the rod pair 17 includes two rods connected with the hemispherical shell 16, the two rods being arranged along front and rear sides of the left notch, and the two rods are connected by means of a bolt 18, so as to clamp the sphere 32 in the hemispherical shell 16.

By means of the above arrangement, the sphere 32 is conveniently fixed in the hemisphere shell 16, and the installation mode is simple and easy to operate.

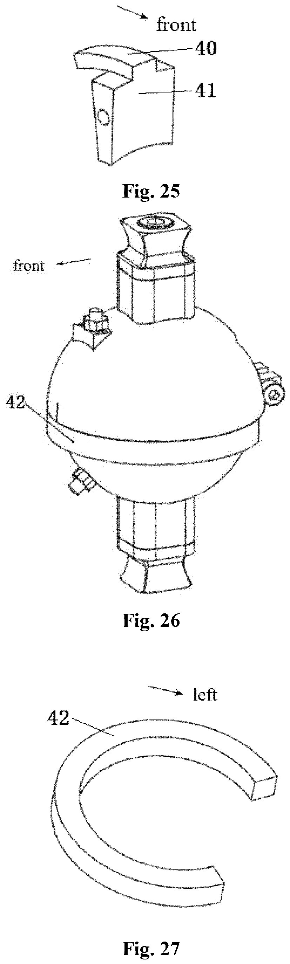

In an embodiment of the present invention, as shown in Figs. 26 and 27, the multi-axis ankle joint prosthesis further includes an auxiliary plate 42, the auxiliary plate 42 is located between the foot connection part and the leg connection part.

In the above arrangement, relative positions of all the components of the ankle joint prosthesis may be conveniently adjusted by means of the auxiliary plate 42, thereby making the ankle joint prosthesis suitable for different wearers, and widening the application range.

In an embodiment of the present invention, the foot connection part A further includes a first connector 4 connected with the lower cover, and the leg connection part B further includes a second connector 8 connected to the upper cover 7.

By means of the above arrangement, the wearer may conveniently install the ankle joint prosthesis between a foot and a leg of the human body by means of the first connector 4 and the second connector 8.

From the above description, it can be seen that the above embodiments of the present invention achieve the following technical effects: by means of the above arrangement, due to the fact that the first boss may slide along the first elongated slide groove, a position of the first slide block assembly relative to the upper cover may be adjusted according to actual requirements; similarly, due to the fact that the second boss may slide along the second elongated slide groove, a position of the second slide block assembly relative to the lower cover may be adjusted according to actual conditions, thus, adjustment may be conducted according to the actual conditions of different wearers, and the multi-axis ankle joint prosthesis is good in adaptability and wider in application range; the first slide block assembly further includes a first protruding block connected with the first boss, and the hemispherical shell assembly further includes a first rail arranged on the hemispherical shell, the first protruding block is in sliding fit with the first rail, so as to make the leg connection part and the first protruding block rotate around a sphere center of the hemispherical shell; the second slide block assembly further includes a second protruding block connected with the second boss, and the sphere assembly further includes a second rail arranged on an outer surface of the sphere, the second protruding block is in sliding fit with the second rail, so as to make the foot connection part and the second protruding block rotate around a sphere center of the sphere, and by using the spherical structure and a sliding fit mode, the movement of the ankle joint prosthesis is closer to that of the ankle joint of the human body, thereby improving the comfort of the wearer.

## Claims

1. A multi-axis ankle joint prosthesis, wherein, the multi-axis ankle joint prosthesis comprises:
a leg connection part (B), comprising an upper cover (7) and a first elongated slide groove (f) provided in the upper cover (7);
a foot connection part (A), comprising a lower cover (5) and a second elongated slide groove (b) provided in the lower cover (5); **characterized in that**,
an upper part (C), comprising a hemispherical shell assembly (F) and a first slide block assembly (E), wherein the first slide block assembly (E) comprises a first boss (22) in sliding fit with the first elongated slide groove (f) and a first threaded rod (21) connected with the first boss (22), the first threaded rod (21) passing through the first elongated slide groove (f) and being detachably installed on the upper cover (7) by a first locking member; the hemispherical shell assembly (F) comprises a hemispherical shell (16), and the leg connection part (B) and the first slide block assembly (E) are rotatably arranged relative to the hemispherical shell (16); and
a lower part (D), comprising a sphere assembly (H) and a second slide block assembly (G), wherein the second slide block assembly (G) comprises a second boss (36) in sliding fit with the second elongated slide groove (b) and a second threaded rod (37) connected with the second boss (36), one end of the second threaded rod (37) penetrating out of the second elongated slide groove (b) and being detachably installed on the lower cover (5) by a second locking member, the sphere assembly (H) comprises a sphere (32) located in the hemispherical shell (16), the sphere (32) being connected with the hemispherical shell (16), and the foot connection part (A) and the second slide block assembly (G) are rotatably arranged relative to the sphere (32);
the first slide block assembly (E) further comprises a first protruding block (23) connected with the first boss (22), and the hemispherical shell assembly (F) further comprises a first rail arranged on the hemispherical shell (16), the first protruding block (23) being in sliding fit with the first rail, so as to make the leg connection part (B) and the first protruding block (23) rotate around a sphere center of the hemispherical shell (16); the hemispherical shell assembly further comprises a first vertical plate (14) and a second vertical plate (15) arranged in parallel front and back on a surface of the hemispherical shell (16), the first rail being formed between the first vertical plate (14) and the second vertical plate (15);
the second slide block assembly (G) further comprises a second protruding block (35) connected with the second boss (36), and the sphere assembly (H) further comprises a second rail arranged on an outer surface of the sphere (32), the second protruding block (35) being in sliding fit with the second rail, so as to make the foot connection part (A) and the second protruding block (35) rotate around a sphere center of the sphere (32); the sphere assembly (H) further comprises a third vertical plate (33) and a fourth vertical plate (34) arranged in parallel left and right on a surface of the sphere (32), the second rail being formed between the third vertical plate (33) and the fourth vertical plate (34);
a left notch (20) is provided in the hemispherical shell (16), the hemispherical shell assembly further comprises a rod pair (17), wherein the rod pair (17) comprises two rods connected with the hemispherical shell (16), the two rods being arranged along front and rear sides of the left notch, and the two rods being connected by a bolt (18), so as to clamp the sphere (32) in the hemispherical shell (16).

2. The multi-axis ankle joint prosthesis according to claim 1, wherein the multi-axis ankle joint prosthesis comprises a first ball (1), a second ball (2), a third ball (3) and an auxiliary plate (42), wherein the first ball (1) is located between the foot connection part (A) and the lower part (D), the first ball (1) contacts with a lower surface of the sphere (32), and the first ball (1) is limited by a first local spherical pit (a) of the lower cover (5); the second ball (2) is located between the leg connection part (B) and the upper part (C), the second ball (2) contacts with the hemispherical shell (16), and the second ball (2) is limited by a second local spherical pit (e) of the upper cover (7) in the leg connection part (B); and the third ball (3) is located between the leg connection part (B) and the upper part (C), the third ball (3) contacts with the hemispherical shell (16), the third ball (3) is limited by a third local spherical pit (g) of the upper cover (7) in the leg connection part (B), and the auxiliary plate (42) is placed between the foot connection part (A) and the leg connection part (B) during angle adjustment, the first rail is an inverted-T-shaped rail; the second rail is an inverted-T-shaped rail; the first locking member and the second locking member are nuts.

3. The multi-axis ankle joint prosthesis according to claim 2, wherein, the foot connection part (A) comprises a first connector (4), wherein the lower cover (5) is of a hollow hemisphere shape with an opening facing upwards, a first stand column (6) is arranged in a center of a lower end of the lower cover (5), and a first threaded blind hole (c) is provided in a center of a lower end of the first stand column (6); the second elongated slide groove (b) is provided in a middle of a right side of the lower cover (5), the first local spherical pit (a) is provided in an inner wall of the lower cover (5), and the first local spherical pit (a) is located on a longitudinal extending line of the second elongated slide groove (b); a first through hole (d) is provided in a center of the first connector (4); and the first connector (4) is fixedly connected with the lower end of the first stand column (6) of the lower cover (5) by means of a bolt.

4. The multi-axis ankle joint prosthesis according to claim 2, wherein, herein the upper cover (7) is in a hollow hemisphere shape with an opening facing downwards, a second stand column (9) is arranged in a center of an upper end of the upper cover (7), and a second threaded blind hole (i) is provided in a center of an upper end of the second stand column (9); a notch (h) is provided in a lower end of a left side of the upper cover (7), and the first elongated slide groove (f) is provided in a right side of the upper cover (7); the second local spherical pit (e) and the third local spherical pit (g) are symmetrically provided in a front side and a rear side of an inner wall of the upper cover (7); a second through hole (j) is provided in a center of a second connector (8); and the second connector (8) is fixedly connected with the upper end of the second stand column (9) of the upper cover (7) by means of a bolt.

5. The multi-axis ankle joint prosthesis according to claim 2, wherein, the upper part (C) comprises a first limiting block (10), a second limiting block (11), a first spring (12) and a second spring (13), wherein a first notch (19) being provided in a lower surface of a right side of the hemispherical shell (16), right ends of two rods of the rod pair (17) are fixedly connected with a lower surface of a left side of the hemispherical shell (16), and a maximum radian of the hemispherical shell (16) slightly exceeds a sphere center; the first limiting block (10) comprises a first transverse block (24) and a first vertical block (25), a lower surface of a right part of the first transverse block (24) being fixedly connected with a top end of the first vertical block (25); the second limiting block (11) comprises a second transverse block (26) and a second vertical block (27), a lower surface of a left part of the second transverse block (26) being fixedly connected with a top end of the second vertical block (27); the first limiting block (10), the first spring (12), the first protruding block (23), the second spring (13) and the second limiting block (11) are sequentially arranged from right to left and located between the first vertical plate (14) and the second vertical plate (15); the first vertical block (25) is fixedly connected with lower right ends of the first vertical plate (14) and the second vertical plate (15); the second vertical block (27) is fixedly connected with upper left ends of the first vertical plate (14) and the second vertical plate (15).

6. The multi-axis ankle joint prosthesis according to claim 2, wherein, the lower part (D) further comprises a third limiting block (28), a fourth limiting block (29), a third spring (30) and a fourth spring (31), wherein; the third limiting block (28) comprises a third transverse block (38) and a third vertical block (39), the third transverse block (38) being fixedly connected with a top end of the third vertical block (39); the fourth limiting block (29) comprises a fourth transverse block (40) and a fourth vertical block (41), the fourth transverse block (40) being fixedly connected with a top end of the fourth vertical block (41); the third limiting block (28), the third spring (30), the second protruding block (35), the fourth spring (31) and the fourth limiting block (29) are sequentially arranged from front to back and located between the third vertical plate (33) and the fourth vertical plate (34); the third vertical block (39) is fixedly connected with rear ends of the third vertical plate (33) and the fourth vertical plate (34); the fourth vertical block (41) is fixedly connected with front ends of the third vertical plate (33) and the fourth vertical plate (34).

7. The multi-axis ankle joint prosthesis according to claim 1, wherein, the hemispherical shell assembly (F) further comprises a first limiting block (10) connected with the first vertical plate (14) and a second limiting block (11) connected with the second vertical plate (15), the first protruding block (23) being located between the first limiting block (10) and the second limiting block (11).

8. The multi-axis ankle joint prosthesis according to claim 1, wherein, the sphere assembly (H) further comprises a third limiting block (28) arranged at a first end of the third vertical plate (33) and a fourth limiting block (29) arranged at a second end of the third vertical plate (33), the second protruding block (35) being located between the third limiting block (28) and the fourth limiting block (29).

9. The multi-axis ankle joint prosthesis according to any one of claims 1, 7 and 8, wherein, the multi-axis ankle joint prosthesis further comprises:
a first ball (1), located between the lower cover and the sphere (32);
a second ball (2), located between the upper cover and the hemispherical shell (16); and
a third ball (3), located between the upper cover and the hemispherical shell (16), the second ball (2) and the third ball (3) being arranged in a front-back direction; and
wherein an inner wall of the lower cover is provided with a first local spherical pit used for limiting the first ball (1), and the upper cover is provided with a second local spherical pit used for limiting the second ball (2) and a third local spherical pit used for limiting the third ball (3); or,
the multi-axis ankle joint prosthesis further comprises an auxiliary plate (42), the auxiliary plate (42) being located between the foot connection part and the leg connection part.

10. The multi-axis ankle joint prosthesis according to any one of claims 1, 7 and 8, wherein, the foot connection part (A) further comprises a first connector (4) connected with the lower cover, and the leg connection part (B) further comprises a second connector (8) connected with the upper cover (7).

## Patentansprüche

1. Mehrachsige Sprunggelenkprothese, wobei die mehrachsige Sprunggelenkprothese umfasst:
einen Beinverbindungsteil (B), der eine obere Abdeckung (7) und eine erste längliche Gleitnut (f) umfasst, die in der oberen Abdeckung (7) vorgesehen ist;
einen Fußverbindungsteil (A), der eine untere Abdeckung (5) und eine zweite längliche Gleitnut (b) umfasst, die in der unteren Abdeckung (5) vorgesehen ist; **gekennzeichnet durch**
einen oberen Teil (C), der eine halbkugelförmige Schalenanordnung (F) und eine erste Gleitblockanordnung (E) umfasst, wobei die erste Gleitblockanordnung (E) einen ersten Vorsprung (22) in Gleitpassung mit der ersten länglichen Gleitnut (f) und eine erste Gewindestange (21) umfasst, die mit dem ersten Vorsprung (22) verbunden ist, wobei die erste Gewindestange (21) durch die erste längliche Gleitnut (f) verläuft und durch ein erstes Verriegelungselement lösbar an der oberen Abdeckung (7) installiert ist; wobei die halbkugelförmige Schalenanordnung (F) eine halbkugelförmige Schale (16) umfasst und der Beinverbindungsteil (B) und die erste Gleitblockanordnung (E) relativ zur halbkugelförmigen Schale (16) drehbar angeordnet sind; und
einen unteren Teil (D), der eine Sphärenanordnung (H) und eine zweite Gleitblockanordnung (G) umfasst, wobei die zweite Gleitblockanordnung (G) einen zweiten Vorsprung (36) in Gleitpassung mit der zweiten länglichen Gleitnut (b) und eine zweite Gewindestange (37) umfasst, die mit dem zweiten Vorsprung (36) verbunden ist, wobei ein Ende der zweiten Gewindestange (37) aus der zweiten länglichen Gleitnut (b) herausragt und durch ein zweites Verriegelungselement lösbar an der unteren Abdeckung (5) installiert ist, wobei die Sphärenanordnung (H) eine in der halbkugelförmigen Schale (16) gelegene Sphäre (32) umfasst, wobei die Sphäre (32) mit der halbkugelförmigen Schale (16) verbunden ist und der Fußverbindungsteil (A) und die zweite Gleitblockanordnung (G) relativ zur Sphäre (32) drehbar angeordnet sind;
wobei die erste Gleitblockanordnung (E) ferner einen ersten vorstehenden Block (23) umfasst, der mit dem ersten Vorsprung (22) verbunden ist, und die halbkugelförmige Schalenanordnung (F) ferner eine erste Schiene umfasst, die an der halbkugelförmigen Schale (16) angeordnet ist, wobei der vorstehende Block (23) in Gleitpassung mit der ersten Schiene steht, sodass sich der Beinverbindungsteil (B) und der erste vorstehende Block (23) um einen Sphärenmittelpunkt der halbkugelförmigen Schale (16) drehen; wobei die halbkugelförmige Schalenanordnung ferner eine erste vertikale Platte (14) und eine zweite vertikale Platte (15) umfasst, die parallel vorne und hinten auf einer Oberfläche der halbkugelförmigen Schale (16) angeordnet sind, wobei die erste Schiene zwischen der ersten vertikalen Platte (14) und der zweiten vertikalen Platte (15) gebildet ist;
wobei die zweite Gleitblockanordnung (G) ferner einen zweiten vorstehenden Block (35) umfasst, der mit dem zweiten Vorsprung (36) verbunden ist, und die Sphärenanordnung (H) ferner eine zweite Schiene umfasst, die auf einer Außenoberfläche der Sphäre (32) angeordnet ist, wobei der zweite vorstehende Block (35) in Gleitpassung mit der zweiten Schiene steht, sodass sich der Fußverbindungsteil (A) und der zweite vorstehende Block (35) um einen Sphärenmittelpunkt der Sphäre (32) drehen; wobei die Sphärenanordnung (H) ferner eine dritte vertikale Platte (33) und eine vierte vertikale Platte (34) umfasst, die parallel links und rechts auf einer Oberfläche der Sphäre (32) angeordnet sind, wobei die zweite Schiene zwischen der dritten vertikalen Platte (33) und der vierten vertikalen Platte (34) gebildet ist;
wobei eine linke Kerbe (20) in der halbkugelförmigen Schale (16) vorgesehen ist, die halbkugelförmige Schalenanordnung ferner ein Stangenpaar (17) umfasst, wobei das Stangenpaar (17) zwei mit der halbkugelförmigen Schale (16) verbundene Stangen umfasst, wobei die zwei Stangen entlang der Vorder- und Rückseite der linken Kerbe angeordnet sind und die zwei Stangen durch einen Bolzen (18) verbunden sind, um die Sphäre (32) in der halbkugelförmigen Schale (16) festzuklemmen.

2. Mehrachsige Sprunggelenkprothese nach Anspruch 1, wobei die mehrachsige Sprunggelenkprothese eine erste Kugel (1), eine zweite Kugel (2), eine dritte Kugel (3) und eine Hilfsplatte (42) umfasst, wobei die erste Kugel (1) zwischen dem Fußverbindungsteil (A) und dem unteren Teil (D) gelegen ist, die erste Kugel (1) in Kontakt mit einer untere Oberfläche der Sphäre (32) steht und die erste Kugel (1) durch eine erste lokale kugelförmige Vertiefung (a) der unteren Abdeckung (5) begrenzt ist; die zweite Kugel (2) zwischen dem Beinverbindungsteil (B) und dem oberen Teil (C) gelegen ist, die zweite Kugel (2) in Kontakt mit der halbkugelförmigen Schale (16) steht und die zweite Kugel (2) durch eine zweite lokale kugelförmige Vertiefung (e) der oberen Abdeckung (7) in dem Beinverbindungsteil (B) begrenzt ist; und die dritte Kugel (3) zwischen dem Beinverbindungsteil (B) und dem oberen Teil (C) gelegen ist, die dritte Kugel (3) in Kontakt mit der halbkugelförmigen Schale (16) steht, die dritte Kugel (3) durch eine dritte lokale kugelförmige Vertiefung (g) der oberen Abdeckung (7) in dem Beinverbindungsteil (B) begrenzt ist, und die Hilfsplatte (42) während einer Winkelanpassung zwischen dem Fußverbindungsteil (A) und dem Beinverbindungsteil (B) platziert ist, die erste Schiene eine umgekehrte T-förmige Schiene ist, die zweite Schiene eine umgekehrt T-förmige Schiene ist; das erste Verriegelungselement und das zweite Verriegelungselement Muttern sind.

3. Mehrachsige Sprunggelenkprothese nach Anspruch 2, wobei der Fußverbindungsteil (A) einen ersten Verbinder (4) umfasst, wobei die untere Abdeckung (5) eine hohle Halbkugelform mit einer nach oben gerichteten Öffnung aufweist, eine erste Standsäule (6) in einer Mitte eines unteren Endes der unteren Abdeckung (5) angeordnet ist und ein erstes Sackloch (c) mit Gewinde in einer Mitte eines unteren Endes der ersten Standsäule (6) vorgesehen ist; die zweite längliche Gleitnut (b) in einer Mitte einer rechten Seite der unteren Abdeckung (5) vorgesehen ist, die erste lokale kugelförmige Vertiefung (a) in einer Innenwand der unteren Abdeckung (5) vorgesehen ist und die erste lokale kugelförmige Vertiefung (a) auf einer sich längs erstreckenden Linie der zweiten länglichen Gleitnut (b) gelegen ist; ein erstes Durchgangsloch (d) in einer Mitte des ersten Verbinders (4) vorgesehen ist; und der erste Verbinder (4) mittels eines Bolzens fest mit dem unteren Ende der ersten Standsäule (6) der unteren Abdeckung (5) verbunden ist.

4. Mehrachsige Sprunggelenkprothese nach Anspruch 2, wobei die obere Abdeckung (7) hierin in einer hohlen Halbkugelform mit einer nach unten gerichteten Öffnung vorliegt, eine zweite Standsäule (9) in einer Mitte eines oberen Endes der oberen Abdeckung (7) angeordnet ist und ein zweites Sackloch (i) mit Gewinde in einer Mitte eines oberen Endes der zweiten Standsäule (9) vorgesehen ist; eine Kerbe (h) in einem unteren Ende einer linken Seite der oberen Abdeckung (7) vorgesehen ist und die erste längliche Gleitnut (f) in einer rechten Seite der oberen Abdeckung (7) vorgesehen ist; die zweite lokale kugelförmige Vertiefung (e) und die dritte lokale kugelförmige Vertiefung (g) symmetrisch auf einer Vorderseite und einer Rückseite einer Innenwand der oberen Abdeckung (7) vorgesehen sind; ein zweites Durchgangsloch (j) in einer Mitte eines zweiten Verbinders (8) vorgesehen ist; und der zweite Verbinder (8) mittels eines Bolzens fest mit dem oberen Ende der zweiten Standsäule (9) der oberen Abdeckung (7) verbunden ist.

5. Mehrachsige Sprunggelenkprothese nach Anspruch 2, wobei der obere Teil (C) einen ersten Begrenzungsblock (10), einen zweiten Begrenzungsblock (11), eine erste Feder (12) und eine zweite Feder (13) umfasst, wobei eine erste Kerbe (19) in einer unteren Oberfläche einer rechten Seite der halbkugelförmigen Schale (16) vorgesehen ist, ein rechtes Ende von zwei Stangen des Stangenpaars (17) fest mit einer unteren Oberfläche einer linken Seite der halbkugelförmigen Schale (16) verbunden sind und ein maximales Bogenmaß der halbkugelförmigen Schale (16) einen Sphärenmittelpunkt geringfügig überschreitet; der erste Begrenzungsblock (10) einen ersten Querblock (24) und einen ersten Vertikalblock (25) umfasst, wobei eine untere Oberfläche eines rechten Teils des ersten Querblocks (24) fest mit einem oberen Ende des ersten Vertikalblocks (25) verbunden ist; der zweite Begrenzungsblock (11) einen zweiten Querblock (26) und einen zweiten Vertikalblock (27) umfasst, wobei eine untere Oberfläche eines linken Teils des zweiten Querblocks (26) fest mit einem oberen Ende des zweiten Vertikalblocks (27) verbunden ist; der erste Begrenzungsblock (10), die erste Feder (12), der erste vorstehende Block (23), die zweite Feder (13) und der zweite Begrenzungsblock (11) nacheinander von rechts nach links angeordnet sind und zwischen der ersten vertikalen Platte (14) und der zweiten vertikalen Platte (15) gelegen sind; der erste Vertikalblock (25) fest mit einem unteren rechten Ende der ersten vertikalen Platte (14) und der zweiten vertikalen Platte (15) verbunden ist; der zweite Vertikalblock (27) fest mit einem oberen linken Ende der ersten vertikalen Platte (14) und der zweiten vertikalen Platte (15) verbunden ist.

6. Mehrachsige Sprunggelenkprothese nach Anspruch 2, wobei der untere Teil (D) ferner einen dritten Begrenzungsblock (28), einen vierten Begrenzungsblock (29), eine dritte Feder (30) und eine vierte Feder (31) umfasst, wobei der dritte Begrenzungsblock (28) einen dritten Querblock (38) und einen dritten Vertikalblock (39) umfasst, wobei der dritte Querblock (38) fest mit einem oberen Ende des dritten Vertikalblocks (39) verbunden ist; der vierte Begrenzungsblock (29) einen vierten Querblock (40) und einen vierten Vertikalblock (41) umfasst, wobei der vierte Querblock (40) fest mit einem oberen Ende des vierten Vertikalblocks (41) verbunden ist; der dritte Begrenzungsblock (28), die dritte Feder (30), der zweite vorstehende Block (35), die vierte Feder (31) und der vierte Begrenzungsblock (29) nacheinander von vorne nach hinten angeordnet sind und zwischen der dritten vertikalen Platte (33) und der vierten vertikalen Platte (34) gelegen sind; der dritte Vertikalblock (39) fest mit einem hinteren Ende der dritten vertikalen Platte (33) und der vierten vertikalen Platte (34) verbunden ist; der vierte Vertikalblock (41) fest mit einem vorderen Ende der dritten vertikalen Platte (33) und der vierten vertikalen Platte (34) verbunden ist.

7. Mehrachsige Sprunggelenkprothese nach Anspruch 1, wobei die halbkugelförmige Schalenanordnung (F) ferner einen ersten Begrenzungsblock (10), der mit der ersten vertikalen Platte (14) verbunden ist, und einen zweiten Begrenzungsblock (11) umfasst, der mit der zweiten vertikalen Platte (15) verbunden ist, wobei der erste vorstehende Block (23) zwischen dem ersten Begrenzungsblock (10) und dem zweiten Begrenzungsblock (11) gelegen ist.

8. Mehrachsige Sprunggelenkprothese nach Anspruch 1, wobei die Sphärenanordnung (H) ferner einen dritten Begrenzungsblock (28), der an einem ersten Ende der dritten vertikalen Platte (33) angeordnet ist, und einen vierten Begrenzungsblock (29) umfasst, der an einem zweiten Ende der dritten vertikalen Platte (33) angeordnet ist, wobei der zweite vorstehende Block (35) zwischen dem dritten Begrenzungsblock (28) und dem vierten Begrenzungsblock (29) gelegen ist.

9. Mehrachsige Sprunggelenkprothese nach einem der Ansprüche 1, 7 und 8, wobei die mehrachsige Sprunggelenkprothese ferner umfasst:
eine erste Kugel (1), die zwischen der unteren Abdeckung und der Sphäre (32) gelegen ist;
eine zweite Kugel (2), die zwischen der oberen Abdeckung und der halbkugelförmigen Schale (16) gelegen ist; und
eine dritte Kugel (3), die zwischen der oberen Abdeckung und der halbkugelförmigen Schale (16) gelegen ist, wobei die zweite Kugel (2) und die dritte Kugel (3) in einer Richtung von vorne nach hinten angeordnet sind; und
wobei eine Innenwand der unteren Abdeckung mit einer ersten lokalen kugelförmigen Vertiefung, die zum Begrenzen der ersten Kugel (1) verwendet wird, versehen ist und die obere Abdeckung mit einer zweiten lokalen kugelförmigen Vertiefung, die zum Begrenzen der zweiten Kugel (2) verwendet wird, und einer dritten lokalen kugelförmigen Vertiefung, die zum Begrenzen der dritten Kugel (3) verwendet wird, versehen ist; oder
die mehrachsige Sprunggelenkprothese ferner eine Hilfsplatte (42) umfasst, wobei die Hilfsplatte (42) zwischen dem Fußverbindungsteil und dem Beinverbindungsteil gelegen ist.

10. Mehrachsige Sprunggelenkprothese nach einem der Ansprüche 1, 7 und 8, wobei der Fußverbindungsteil (A) ferner einen ersten Verbinder (4) umfasst, der mit der unteren Abdeckung verbunden ist, und der Beinverbindungsteil (B) ferner einen zweiten Verbinder (8) umfasst, der mit der oberen Abdeckung (7) verbunden ist.

## Revendications

1. Prothèse d'articulation de cheville à axes multiples, la prothèse d'articulation de cheville à axes multiples comprenant :
une partie de liaison de jambe (B), comprenant un couvercle supérieur (7) et une première rainure de coulissement allongée (f) prévue dans le couvercle supérieur (7) ;
une partie de liaison de pied (A), comprenant un couvercle inférieur (5) et une seconde rainure de coulissement allongée (b) prévue dans le couvercle inférieur (5) ; **caractérisée en ce que**
une partie supérieure (C), comprenant un ensemble coque hémisphérique (F) et un premier ensemble bloc coulissant (E), dans laquelle le premier ensemble bloc coulissant (E) comprend un premier bossage (22) en ajustement coulissant avec la première rainure de coulissement allongée (f) et une première tige filetée (21) reliée au premier bossage (22), la première tige filetée (21) traversant la première rainure de coulissement allongée (f) et étant installée de façon amovible sur le couvercle supérieur (7) par un premier organe de verrouillage ; l'ensemble coque hémisphérique (F) comprend une coque hémisphérique (16) et la partie de liaison de jambe (B) et le premier ensemble bloc coulissant (E) sont agencés de manière rotative par rapport à la coque hémisphérique (16) ; et
une partie inférieure (D), comprenant un ensemble sphère (H) et un second ensemble bloc coulissant (G), dans laquelle le second ensemble bloc coulissant (G) comprend un second bossage (36) en ajustement coulissant avec la seconde rainure de coulissement allongée (b) et une seconde tige filetée (37) reliée au second bossage (36), une extrémité de la seconde tige filetée (37) pénétrant hors de la seconde rainure de coulissement allongée (b) et étant installée de manière amovible sur le couvercle inférieur (5) par un second organe de verrouillage, l'ensemble sphère (H) comprend une sphère (32) située dans la coque hémisphérique (16), la sphère (32) étant reliée à la coque hémisphérique (16) et la partie de liaison de pied (A) et le second ensemble bloc coulissant (G) sont agencés de manière rotative par rapport à la sphère (32) ;
le premier ensemble bloc coulissant (E) comprend en outre un premier bloc en saillie (23) relié au premier bossage (22) et l'ensemble coque hémisphérique (F) comprend en outre un premier rail agencé sur la coque hémisphérique (16), le premier bloc en saillie (23) étant en ajustement coulissant avec le premier rail, de manière à faire tourner la partie de liaison de jambe (B) et le premier bloc en saillie (23) autour d'un centre de sphère de la coque hémisphérique (16) ; l'ensemble coque hémisphérique comprend en outre une première plaque verticale (14) et une deuxième plaque verticale (15) agencées parallèlement à l'avant et à l'arrière sur une surface de la coque hémisphérique (16), le premier rail étant formé entre la première plaque verticale (14) et la deuxième plaque verticale (15) ;
le second ensemble bloc coulissant (G) comprend en outre un second bloc en saillie (35) relié au second bossage (36) et l'ensemble sphère (H) comprend en outre un second rail agencé sur une surface externe de la sphère (32), le second bloc en saillie (35) étant en ajustement coulissant avec le second rail, de manière à faire tourner la partie de liaison de pied (A) et le second bloc en saillie (35) autour d'un centre de sphère de la sphère (32) ; l'ensemble sphère (H) comprend en outre une troisième plaque verticale (33) et une quatrième plaque verticale (34) agencées parallèlement à gauche et à droite sur une surface de la sphère (32), le second rail étant formé entre la troisième plaque verticale (33) et la quatrième plaque verticale (34) ;
une encoche gauche (20) est prévue dans la coque hémisphérique (16), l'ensemble coque hémisphérique comprend en outre une paire de tiges (17), dans laquelle la paire de tiges (17) comprend deux tiges reliées à la coque hémisphérique (16), les deux tiges étant agencées le long de côtés avant et arrière de l'encoche gauche et les deux tiges étant reliées par un boulon (18), de manière à serrer la sphère (32) dans la coque hémisphérique (16).

2. Prothèse d'articulation de cheville à axes multiples selon la revendication 1, dans laquelle la prothèse d'articulation de cheville à axes multiples comprend une première bille (1), une deuxième bille (2), une troisième bille (3) et une plaque auxiliaire (42), dans laquelle la première bille (1) est située entre la partie de liaison de pied (A) et la partie inférieure (D), la première bille (1) entre en contact avec une surface inférieure de la sphère (32) et la première bille (1) est limitée par un premier creux sphérique local (a) du couvercle inférieur (5) ; la deuxième bille (2) est située entre la partie de liaison de jambe (B) et la partie supérieure (C), la deuxième bille (2) entre en contact avec la coque hémisphérique (16) et la deuxième bille (2) est limitée par un deuxième creux sphérique local (e) du couvercle supérieur (7) dans la partie de liaison de jambe (B) ; et la troisième bille (3) est située entre la partie de liaison de jambe (B) et la partie supérieure (C), la troisième bille (3) entre en contact avec la coque hémisphérique (16), la troisième bille (3) est limitée par un troisième creux sphérique local (g) du couvercle supérieur (7) dans la partie de liaison de jambe (B) et la plaque auxiliaire (42) est placée entre la partie de liaison de pied (A) et la partie de liaison de jambe (B) pendant le réglage d'angle, le premier rail est un rail en T inversé ; le second rail est un rail en T inversé ; le premier organe de verrouillage et le second organe de verrouillage sont des écrous.

3. Prothèse d'articulation de cheville à axes multiples selon la revendication 2, dans laquelle la partie de liaison de pied (A) comprend un premier raccord (4), dans laquelle le couvercle inférieur (5) est en forme d'hémisphère creux avec une ouverture tournée vers le haut, une première colonne de support (6) est agencée au centre d'une extrémité inférieure du couvercle inférieur (5) et un premier trou borgne fileté (c) est prévu au centre d'une extrémité inférieure de la première colonne de support (6) ; la seconde rainure de coulissement allongée (b) est prévue au milieu d'un côté droit du couvercle inférieur (5), le premier creux sphérique local (a) est prévu dans une paroi interne du couvercle inférieur (5) et le premier creux sphérique local (a) est situé sur une ligne d'extension longitudinale de la seconde rainure de coulissement allongée (b) ; un premier trou traversant (d) est prévu au centre du premier raccord (4) ; et le premier raccord (4) est relié de manière fixe à l'extrémité inférieure de la première colonne de support (6) du couvercle inférieur (5) au moyen d'un boulon.

4. Prothèse d'articulation de cheville à axes multiples selon la revendication 2, dans laquelle le couvercle supérieur (7) des présentes est en forme d'hémisphère creux avec une ouverture tournée vers le bas, une seconde colonne de support (9) est agencée au centre d'une extrémité supérieure du couvercle supérieur (7) et un second trou borgne fileté (i) est prévu au centre d'une extrémité supérieure de la seconde colonne de support (9) ; une encoche (h) est prévue dans une extrémité inférieure d'un côté gauche du couvercle supérieur (7) et la première rainure de coulissement allongée (f) est prévue dans un côté droit du couvercle supérieur (7) ; le deuxième creux sphérique local (e) et le troisième creux sphérique local (g) sont prévus symétriquement dans un côté avant et un côté arrière d'une paroi interne du couvercle supérieur (7) ; un second trou traversant (j) est prévu au centre d'un second raccord (8) ; et le second raccord (8) est relié de manière fixe à l'extrémité supérieure de la seconde colonne de support (9) du couvercle supérieur (7) au moyen d'un boulon.

5. Prothèse d'articulation de cheville à axe multiples selon la revendication 2, dans laquelle la partie supérieure (C) comprend un premier bloc de limitation (10), un deuxième bloc de limitation (11), un premier ressort (12) et un deuxième ressort (13), dans laquelle une première encoche (19) étant prévue dans une surface inférieure d'un côté droit de la coque hémisphérique (16), les extrémités droites de deux tiges de la paire de tiges (17) sont reliées de manière fixe à une surface inférieure d'un côté gauche de la coque hémisphérique (16) et un radian maximum de la coque hémisphérique (16) dépasse légèrement un centre de sphère ; le premier bloc de limitation (10) comprend un premier bloc transversal (24) et un premier bloc vertical (25), une surface inférieure d'une partie droite du premier bloc transversal (24) étant reliée de manière fixe à une extrémité supérieure du premier bloc vertical (25) ; le deuxième bloc de limitation (11) comprend un deuxième bloc transversal (26) et un deuxième bloc vertical (27), une surface inférieure d'une partie gauche du deuxième bloc transversal (26) étant reliée de manière fixe à une extrémité supérieure du deuxième bloc vertical (27) ; le premier bloc de limitation (10), le premier ressort (12), le premier bloc en saillie (23), le deuxième ressort (13) et le deuxième bloc de limitation (11) sont agencés séquentiellement de droite à gauche et situés entre la première plaque verticale (14) et la deuxième plaque verticale (15) ; le premier bloc vertical (25) est relié de manière fixe aux extrémités inférieures droites de la première plaque verticale (14) et de la deuxième plaque verticale (15) ; le deuxième bloc vertical (27) est relié de manière fixe aux extrémités supérieures gauches de la première plaque verticale (14) et de la deuxième plaque verticale (15).

6. Prothèse d'articulation de cheville à axes multiples selon la revendication 2, dans laquelle la partie inférieure (D) comprend en outre un troisième bloc de limitation (28), un quatrième bloc de limitation (29), un troisième ressort (30) et un quatrième ressort (31), dans laquelle le troisième bloc de limitation (28) comprend un troisième bloc transversal (38) et un troisième bloc vertical (39), le troisième bloc transversal (38) étant relié de manière fixe à une extrémité supérieure du troisième bloc vertical (39) ; le quatrième bloc de limitation (29) comprend un quatrième bloc transversal (40) et un quatrième bloc vertical (41), le quatrième bloc transversal (40) étant relié de manière fixe à une extrémité supérieure du quatrième bloc vertical (41) ; le troisième bloc de limitation (28), le troisième ressort (30), le second bloc en saillie (35), le quatrième ressort (31) et le quatrième bloc de limitation (29) sont agencés séquentiellement d'avant en arrière et situés entre la troisième plaque verticale (33) et la quatrième plaque verticale (34) ; le troisième bloc vertical (39) est relié de manière fixe aux extrémités arrière de la troisième plaque verticale (33) et de la quatrième plaque verticale (34) ; le quatrième bloc vertical (41) est relié de manière fixe aux extrémités avant de la troisième plaque verticale (33) et de la quatrième plaque verticale (34).

7. Prothèse d'articulation de cheville à axes multiples selon la revendication 1, dans laquelle l'ensemble coque hémisphérique (F) comprend en outre un premier bloc de limitation (10) relié à la première plaque verticale (14) et un deuxième bloc de limitation (11) relié à la deuxième plaque verticale (15), le premier bloc en saillie (23) étant situé entre le premier bloc de limitation (10) et le deuxième bloc de limitation (11).

8. Prothèse d'articulation de cheville à axes multiples selon la revendication 1, dans laquelle l'ensemble sphère (H) comprend en outre un troisième bloc de limitation (28) agencé à une première extrémité de la troisième plaque verticale (33) et un quatrième bloc de limitation (29) agencé à une seconde extrémité de la troisième plaque verticale (33), le second bloc en saillie (35) étant situé entre le troisième bloc de limitation (28) et le quatrième bloc de limitation (29).

9. Prothèse d'articulation de cheville à axes multiples selon l'une quelconque des revendications 1, 7 et 8, dans laquelle la prothèse d'articulation de cheville à axes multiples comprend en outre :
une première bille (1), située entre le couvercle inférieur et la sphère (32) ;
une deuxième bille (2), située entre le couvercle supérieur et la coque hémisphérique (16) ; et
une troisième bille (3), située entre le couvercle supérieur et la coque hémisphérique (16), la deuxième bille (2) et la troisième bille (3) étant agencées dans une direction avant-arrière ; et
dans laquelle une paroi interne du couvercle inférieur est pourvue d'un premier creux sphérique local utilisé pour limiter la première bille (1) et le couvercle supérieur est pourvu d'un deuxième creux sphérique local utilisé pour limiter la deuxième bille (2) et d'un troisième creux sphérique local utilisé pour limiter la troisième bille (3) ; ou
la prothèse d'articulation de cheville à axes multiples comprend en outre une plaque auxiliaire (42), la plaque auxiliaire (42) étant située entre la partie de liaison de pied et la partie de liaison de jambe.

10. Prothèse d'articulation de cheville à axes multiples selon l'une quelconque des revendications 1, 7 et 8, dans laquelle la partie de liaison de pied (A) comprend en outre un premier raccord (4) relié au couvercle inférieur et la partie de liaison de jambe (B) comprend en outre un second raccord (8) relié au couvercle supérieur (7).
